# EUROPEAN PATENT APPLICATION

(11) **EP 4 813 465 A1**
(43) Date of publication of application: **30.09.2026**
(21) Application number: 26167684.5
(22) Date of filing: 25.03.2026
(51) Int. Cl.: A61Q 19/08, A61K 8/67, A61K 8/365

(54) **COSMETIC COMPOSITION COMPRISING POLYHYDROXY ACID STABILIZED BY VITAMIN B5**

(30) Priority: 28.03.2025 KR 20250040023
(71) Applicant: Amorepacific Corporation, Seoul 04386 (KR)
(72) Inventor: Song, Jung Ho, 17074 Yongin-si, Gyeonggi-do (KR)
(74) Representative: Schön, Christoph

(57) **Abstract**

One aspect of the present disclosure relates to a cosmetic composition including a high content of polyhydroxy acid (PHA). More specifically, one aspect of the present disclosure relates to a cosmetic composition comprising vitamin B5 together with a high content of PHA, wherein the high content of PHA is stabilized without dissociation by vitamin B5.

## Description

### [Technical Field]

### Cross-citation with Related Applications

This application claims the benefit of priority to Korean Patent Application No. 10-2025-0040023, filed March 28, 2025.

### Technical Field

One aspect of the present disclosure relates to a cosmetic composition comprising a high content of polyhydroxy acid (PHA). More specifically, one aspect of the present disclosure relates to a cosmetic composition comprising vitamin B5 together with a high content of PHA, wherein the high content of PHA is stabilized without dissociation by vitamin B5.

### [Background Art]

Polyhydroxy acid (PHA) is an organic carboxylic acid with two or more hydroxy groups attached to the chain of an aliphatic compound or an aromatic compound. The PHA contains hydroxyl groups at various positions including the alpha position. In PHA, the hydroxyl group is neutral, and the carboxyl group indicates acidity.

PHA included in a cosmetic composition has the effect of improving the skin, by lowering the ion bonding energy of skin keratinocytes to remove excess dead skin cells or promoting the formation of new cells, thereby shortening the cycle of the keratinization process that becomes longer as the skin ages and promoting the activity of skin cells. Accordingly, as the content of PHA included in a cosmetic composition increases, its efficacy also increases significantly, but it has the disadvantage of causing skin irritation when used in large quantities, and thus, its use in cosmetics is extremely limited.

In addition, as the content of PHA in a cosmetic composition increases, it may be difficult to control pH. A high content of PHA tends to dissociate over time, causing the pH to decrease. If the pH decreases, it may be difficult to use as a cosmetic because the irritation to the skin increases.

Accordingly, a method of using a neutralizer to stabilize the cosmetic composition by preventing it from becoming highly acidic due to a high content of PHA has been developed. If a strong base is used as the neutralizer, the amount of neutralizer used may be reduced, but the irritation to the skin may still exist. If a weak base is used as the neutralizer, a large amount of neutralizer may be used, which may cause irritation and reduce the feeling of use.

In this way, cosmetic compositions comprising a high content of PHA have high acidity and increased stickiness, so even if they are stabilized with a neutralizer, they have limitations in using them as actual cosmetic compositions due to poor feeling of use.

Therefore, there is a need for the development of technology that may improve the feeling of use while stabilizing cosmetic compositions comprising a high content of PHA.

### [Disclosure]

### [Technical Problem]

In order to solve the above problems, the present inventors have studied a composition having improved stability and feeling of use, comprising a high content of polyhydroxy acid (PHA) and vitamin B5, and have completed one aspect of the present disclosure.

Therefore, it is an object of one aspect of the present disclosure to provide a cosmetic composition having improved stability and feeling of use.

### [Technical Solution]

According to the first aspect of one aspect of the present disclosure, there is provided a cosmetic composition comprising polyhydroxy acid (PHA) and vitamin B5, wherein the PHA is included in an amount of 15% by weight or more based on the total weight of the cosmetic composition, and the vitamin B5 is used for stabilizing PHA by preventing dissociation of PHA.

In addition, the cosmetic composition is provided, wherein the vitamin B5 is included in an amount of 4 to 6% by weight based on the total weight of the cosmetic composition.

In addition, the cosmetic composition is provided, wherein the pH of the cosmetic composition is 3.9 or more.

In addition, the cosmetic composition is provided, wherein the pH of the cosmetic composition is maintained at 3.9 or more for 4 weeks or more.

In addition, the cosmetic composition is provided, which does not comprise a buffer.

In addition, the cosmetic composition is provided, wherein the cosmetic composition is formulated into a skin, lotion, cream, serum, emulsion, essence, powder, foundation, spray, sunscreen, mask pack or gel.

In addition, the cosmetic composition is provided, wherein the cosmetic composition further comprises one or more additives selected from the group consisting of a neutralizer, a surfactant, a moisturizer, a preservative, an ultraviolet absorber, a pigment, an inorganic salt, and an organic acid salt.

In addition, the cosmetic composition is provided, wherein the neutralizer includes one or more selected from the group consisting of tromethamine, sodium hydroxide, and potassium hydroxide.

### [Advantageous Effects]

The cosmetic composition according to one aspect of the present disclosure may secure the stability of the cosmetic composition and improve the feeling of use by comprising vitamin B5 together with a high content of PHA.

### [Description of Drawings]

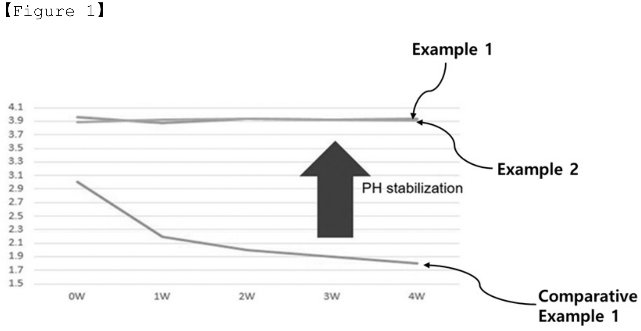

FIG. 1 is a graph illustrating the results of a pH stability test for cosmetic compositions prepared in Example 1, Example 2, and Comparative Example 1.

### [Best Mode]

The embodiments provided according to can be all achieved by the following description. It should be understood that the following description describes preferred embodiments of one aspect of the present disclosure and one aspect of the present disclosure is not necessarily limited thereto.

The term "stabilizing PHA" as used in the present specification means preventing dissociation of PHA included in a cosmetic composition. Specifically, in order for the PHA to not dissociate, the pH may be maintained within an appropriate range, and thus, it means that the PHA is stabilized without dissociation as the pH of the cosmetic composition is maintained within an appropriate range by vitamin B5.

In one embodiment of one aspect of the present disclosure, the cosmetic composition comprises polyhydroxy acid (PHA) and vitamin B5, wherein the PHA is included in an amount of 15% by weight or more based on the total weight of the cosmetic composition, and the vitamin B5 may be for stabilizing PHA by preventing dissociation of PHA.

The PHA may function to improve the skin by promoting the activity of skin cells through exfoliation, and thus, the higher the content of PHA in a cosmetic composition, the more advantageous it may be in terms of skin improvement. The content of the PHA included in the cosmetic composition may be 15% by weight or more based on the total weight of the cosmetic composition. Specifically, the content of the PHA may be 15% by weight or more, 16% by weight or more, 17% by weight or more, 18% by weight or more, 19% by weight or more, 20% by weight or more, 21% by weight or more, 22% by weight or more, 23% by weight or more, 24% by weight or more, 25% by weight or more, 26% by weight or more, 27% by weight or more, 28% by weight or more, 29% by weight or more, 30% by weight or more, 31% by weight or more, 32% by weight or more, 33% by weight or more, 34% by weight or more, or 35% by weight or more. The upper limit of the PHA content is not particularly limited, but may be 45% by weight or less, 44% by weight or less, 43% by weight or less, 42% by weight or less, 41% by weight or less, or 40% by weight or less, taking into consideration skin irritation.

In addition, the PHA dissociates over time, which causes the pH of the cosmetic composition to decrease, and thus, the acidity tends to increase. Specifically, as shown in following Formula (1), PHA tends to gradually dissociate from δ-gluconolactone to D-gluconic acid and gluconate over time, and thus, the pH of the cosmetic composition may decrease and the acidity may increase:

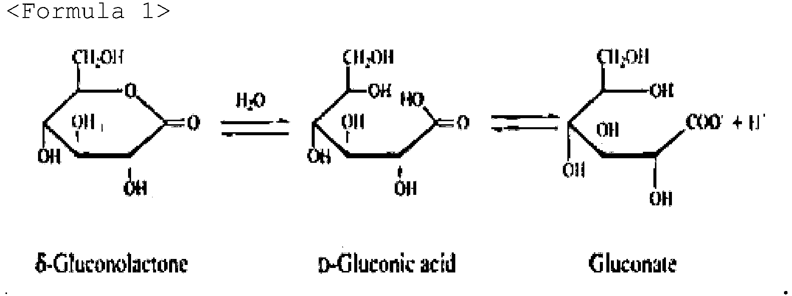

In order to prevent such dissociation of PHA, it is necessary to maintain the pH of the cosmetic composition at 3.9 or more. Under conditions of pH 3.9 or more, PHA may maintain stability. Vitamin B5 may be included to maintain the pH of the cosmetic composition at 3.9 or more. Vitamin B5 may play a role in preventing the dissociation of PHA while neutralizing the reduced pH caused by some of the dissociated PHA.

In one embodiment of one aspect of the present disclosure, the vitamin B5 may be included in an amount of 4 to 6% by weight based on the total weight of the cosmetic composition.

The vitamin B5 is one of the water-soluble vitamins belonging to the vitamin B complex. The vitamin B5 may be easily absorbed through the skin to play a role in providing moisture to the skin and protecting damaged skin.

In addition, if the content of the vitamin B5 is less than 4% by weight based on the total weight of the cosmetic composition, the pH stabilization effect may be minimal, and if it exceeds 6% by weight, the feeling of use may deteriorate. Specifically, the content of the vitamin B5 may be 4% by weight or more, 4.1% by weight or more, 4.2% by weight or more, 4.3% by weight or more, 4.4% by weight or more, 4.5% by weight or more, 4.6% by weight or more, 4.7% by weight or more, 4.8% by weight or more, 4.9% by weight or more, or 5% by weight or more, and may be 6% by weight or less, 5.9% by weight or less, 5.8% by weight or less, 5.7% by weight or less, 5.6% by weight or less, 5.5% by weight or less, 5.4% by weight or less, 5.3% by weight or less, 5.2% by weight or less, or 5.1% by weight or less.

In one embodiment of one aspect of the present disclosure, the pH of the cosmetic composition may be 3.9 or more.

If the pH of the cosmetic composition is less than 3.9, the PHA tends to dissociate, which causes the pH to decrease and the acidity to increase, and thus, skin irritation may increase. Specifically, the pH of the cosmetic composition may be 3.9 or more, 4 or more, 4.1 or more, 4.2 or more, 4.3 or more, 4.4 or more, 4.5 or more, 4.6 or more, 4.7 or more, 4.8 or more, 4.9 or more, 5 or more, 5.1 or more, 5.2 or more, 5.3 or more, 5.4 or more, or 5.5 or more. In addition, the pH of the cosmetic composition may be 6 or less, 5.9 or less, 5.8 or less, 5.7 or less, or 5.6 or less, taking into consideration the feeling of use.

In one embodiment of one aspect of the present disclosure, the pH of the cosmetic composition may be maintained at 3.9 or more for 4 weeks or more.

The cosmetic composition comprises a high content of PHA together with vitamin B5, which causes the pH to be maintained at a certain level even when stored for a long period of time, and thus, stability may be secured.

In this way, as the long-term storage stability of the cosmetic composition is secured, the effectiveness of the product may be maintained, the appearance and feeling of use of the product may be maintained, and economic feasibility may be secured. The effectiveness of the product means that it may continuously provide consistent effects throughout the period of use by preventing the high content of active ingredient PHA from deteriorating or decomposing.

In one embodiment of one aspect of the present disclosure, the cosmetic composition may not comprise a buffer (non-buffered).

The cosmetic composition may secure pH stability due to vitamin B5, and thus, it does not need to comprise a separate buffer. Therefore, stability degradation and skin irritation due to the buffer may be prevented. In general, buffers included in cosmetic compositions serve to maintain pH, but may interact with other active ingredients, which reduces the efficacy of the product or negatively affects long-term stability, thereby reducing stability. In addition, certain buffer ingredients may be irritating to the skin and, for example, may cause allergies in users with sensitive skin.

In one embodiment of one aspect of the present disclosure, the cosmetic composition may comprise a neutralizer.

The neutralizer may stabilize the cosmetic composition by preventing it from being excessively acidified by a high content of PHA, and may stabilize the cosmetic composition even when included in a small amount together with vitamin B5. In addition, since only a small amount of neutralizer is used, the feeling of use may not deteriorate.

Specifically, the neutralizer may include one or more selected from the group consisting of tromethamine, sodium hydroxide, and potassium hydroxide. Considering the stabilizing effect by combined use with vitamin B5, the neutralizer may be tromethamine.

In addition, the neutralizer may be included in an amount of 1 to 5% by weight based on the total weight of the cosmetic composition. The content range of the neutralizer is set in consideration of the degree to which the cosmetic composition may be stabilized while ensuring a feeling of use through combined use with vitamin B5 in a cosmetic composition comprising 15% by weight or more of PHA. If the content of the neutralizer is less than 1% by weight, the stabilizing effect may be minimal, and if it exceeds 5% by weight, the feeling of use may be degraded. Specifically, the content of the neutralizer may be 1% by weight or more, 1.5% by weight or more, 2% by weight or more, 2.5% by weight or more, 3% by weight or more, or 3.5% by weight or more, and may be 5% by weight or less, 4.9% by weight or less, 4.8% by weight or less, 4.7% by weight or less, 4.6% by weight or less, 4.5% by weight or less, 4.4% by weight or less, 4.3% by weight or less, 4.2% by weight or less, or 4.1% by weight or less.

In one embodiment of one aspect of the present disclosure, the cosmetic composition is formulated into one or more selected from the group consisting of a skin, a lotion, a cream, a serum, an emulsion, an essence, a powder, a foundation, a spray, a sunscreen, a mask pack, or a gel.

In one embodiment of one aspect of the present disclosure, the cosmetic composition may further comprise one or more additives selected from the group consisting of a surfactant, a moisturizer, a preservative, an ultraviolet absorber, a pigment, an antioxidant, a viscosity modifier, a fragrance, an inorganic salt, and an organic acid salt.

The surfactant may include surfactants generally used in cosmetic compositions, such as, for example, nonionic surfactants such as polyoxyethylene alkyl ethers, polyoxyethylene fatty acid esters, polyoxyethylene sorbitan fatty acid esters, glycerin fatty acid esters, polyglycerin fatty acid esters, polyoxyethylene glycerin fatty acid esters, polyoxyethylene hydrogenated castor oil and polyoxyethylene sorbitol fatty acid esters; anionic surfactants represented by fatty acid soaps such as sodium stearate and triethanolamine palmitate; cationic surfactants; and amphoteric surfactants.

The moisturizer may include moisturizers commonly used in cosmetics, such as, for example, sorbitol, xylitol, glycerin, maltitol, propylene glycol, 1,3-butylene glycol, 1,4-butylene glycol, sodium pyrrolidonecarboxylate, lactic acid, sodium lactate and polyethylene glycol.

The preservative may include preservatives commonly used in cosmetics, such as, for example, paraoxybenzoic acid alkyl ester, sodium benzoate and potassium sorbate.

The ultraviolet absorber may include ultraviolet absorbers commonly used in cosmetics, such as, for example, para-aminobenzoic acid-based ultraviolet absorbers, anthranil-based ultraviolet absorbers, salicylic acid-based ultraviolet absorbers, cinnamic acid-based ultraviolet absorbers and benzophenone-based ultraviolet absorbers.

The pigment may include pigments commonly used in cosmetics, such as, for example, inorganic pigments such as red iron oxide, yellow iron oxide or black iron oxide; Tar pigments such as Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206 and Orange No. 207; natural pigments such as carminic acid, laccaic acid, brazilin and crocin.

The antioxidant may include, for example, ascorbic acid, and salts and derivatives thereof, such as ascorbyl glucoside, ethyl ascorbyl ether, ascorbyl ether, ascorbyl phosphate, and salts thereof, caffeic acid and salts thereof, and the like.

The viscosity modifier may include, for example, lauryl hydroxyl sultaine, disodium cocodiamphoacetate, or a combination thereof.

The inorganic salt or organic acid salt may include alkali metal salts, alkaline earth metal salts or aluminum salts of inorganic acids such as hydrochloric acid, sulfuric acid and nitric acid; oxycarboxylic acids such as citric acid, tartaric acid, lactic acid and malic acid; carboxylic acids such as formic acid, acetic acid and sorbic acid; or aromatic carboxylic acids such as salicylic acid and benzoic acid.

Hereinafter, preferred examples will be provided to help understanding one aspect of the present disclosure, but the following examples are only for illustrating one aspect of the present disclosure, and it will be apparent to those skilled in the art that various changes and modifications may be made within the scope and technical spirit of one aspect of the present disclosure, and it is obvious that such changes and modifications fall within the scope of the appended claims.

In the following examples and comparative examples, cosmetic compositions were prepared according to the compositions shown in Table 1 below.

**[Table 1]**

| Unit: % by weight | | Exampl e 1 | Exampl e 2 | Exampl e 3 | Exampl e 4 | Exampl e 5 | Exampl e 6 | Exampl e 7 | Compar ative Exampl e 1 | Compar ative Exampl e 2 |
|---|---|---|---|---|---|---|---|---|---|---|
| 1 | PHA | 15 | 15 | 15 | 15 | 15 | 20 | 30 | 15 | 15 |
| 2 | Vitamin B5 | 4 | 5 | 6 | 3 | 7 | 4 | 4 | - | 4 |
| 3 | Glycerin | 9 | 9 | 9 | 9 | 9 | 12 | 15 | 9 | 9 |
| 4 | Tromethamine | 4 | 4 | 4 | 4 | 4 | 5 | 5 | 4 | - |
| 5 | Betaine | 2 | 2 | 2 | 2 | 2 | 2 | 2 | 2 | 2 |
| 6 | PPG-13-decyltetradece th-24 | 0.3 | 0.3 | 0.3 | 0.3 | 0.3 | 0.3 | 0.3 | 0.3 | 0.3 |
| 7 | Sodium hyaluronate | 0.1 | 0.1 | 0.1 | 0.1 | 0.1 | 0.1 | 0.1 | 0.1 | 0.1 |
| 8 | 1,2-Hexanediol | 1 | 1 | 1 | 1 | 1 | 1 | 1 | 1 | 1 |
| 9 | Sodium metaphosphate | 0.04 | 0.04 | 0.04 | 0.04 | 0.04 | 0.04 | 0.04 | 0.04 | 0.04 |
| 1 0 | D.I. Water | Up to 100 | Up to 100 | Up to 100 | Up to 100 | Up to 100 | Up to 100 | Up to 100 | Up to 100 | Up to 100 |

### Example 1

A cosmetic composition was prepared by mixing the raw materials having the composition shown in Table 1 above. 15% by weight of polyhydroxy acid (PHA) and 4% by weight of vitamin B5 were used. In addition, 4% by weight of tromethamine was used as a neutralizer.

### Example 2

A cosmetic composition was prepared in the same manner as in Example 1, except that the content of vitamin B5 was 5% by weight.

### Example 3

A cosmetic composition was prepared in the same manner as in Example 1, except that the content of vitamin B5 was 6% by weight.

### Example 4

A cosmetic composition was prepared in the same manner as in Example 1, except that the content of vitamin B5 was 3% by weight.

### Example 5

A cosmetic composition was prepared in the same manner as in Example 1, except that the content of vitamin B5 was 7% by weight.

### Example 6

A cosmetic composition was prepared in the same manner as in Example 1, except that the content of PHA was 20% by weight.

### Example 7

A cosmetic composition was prepared in the same manner as in Example 1, except that the content of PHA was 30% by weight.

### Comparative Example 1

A cosmetic composition was prepared in the same manner as in Example 1, except that vitamin B5 was not used.

### Comparative Example 2

A cosmetic composition was prepared in the same manner as in Example 1, except that tromethamine, which is a neutralizer, was not used.

### Experimental Example 1: Evaluation of pH stability

An experiment was conducted on the pH stability of the cosmetic compositions prepared in the examples and comparative examples. The cosmetic compositions were stored in a cycling chamber (repeatedly changing between 40°C and -15°C every 12 hours) at room temperature (RT), 30°C, 45°C, 60°C, 4°C and -15°C for 4 weeks or more and the pH was measured every week, and the results are as shown in Table 2 below.

**[Table 2]**

| pH | ow | 1W | 2W | 3W | 4W |
|---|---|---|---|---|---|
| Example 1 | 3.96 | 3.88 | 3.94 | 3.92 | 3.94 |
| Example 2 | 3.99 | 4.01 | 4.00 | 4.01 | 4.00 |
| Example 3 | 4.05 | 4.07 | 4.07 | 4.06 | 4.07 |
| Example 4 | 3.88 | 3.75 | 3.63 | 3.59 | 3.49 |
| Example 5 | 4.04 | 4.04 | 4.03 | 4.04 | 4.04 |
| Example 6 | 3.89 | 3.92 | 3.94 | 3.92 | 3.91 |
| Example 7 | 3.91 | 3.92 | 3.94 | 9.92 | 3.92 |
| Comparative Example 1 | 3.91 | 3.71 | 3.61 | 3.55 | 3.32 |
| Comparative Example 2 | 4.04 | 3.88 | 3.78 | 3.61 | 3.51 |

FIG. 1 is a graph illustrating the results of a pH stability test for cosmetic compositions prepared in Example 1, Example 2, and Comparative Example 1.

Referring to FIG. 1 and Table 1 above, it can be seen that although Examples 1 and 2 comprise a high content of PHA, the difference in pH between the initial (0W) and the 4th week (4W) of storage is minimal. On the other hand, it can be seen that Comparative Example 1 showed a remarkable decrease in pH at 4 weeks (4W) compared to the initial (0W) of storage, indicating that acidity increased and that many side effects would occur when applied to the skin. It can be seen that Examples 1 and 2 comprise vitamin B5 together with a high content of PHA, and thus, vitamin B5 functions to stabilize the pH of a cosmetic composition comprising a high content of PHA.

In addition, Examples 1 to 5 are cosmetic compositions comprising different contents of vitamin B5, and while Examples 1 to 3 exhibited pH stability, Example 4 showed a rapid decrease in pH and an increase in acidity over time, indicating that the pH stabilization functionality was reduced due to a decrease in the content of vitamin B5.

### Experimental Example 2: Sensory (feeling of use) Evaluation

A sensory evaluation of the cosmetic compositions prepared in the examples and comparative examples was conducted on 20 women aged 25 to 35 years, and the results are shown in Table 3 below.

### [Sensory Evaluation Criteria]

⊚: Very excellent
○: Excellent
△: Average
X: Bad

**[Table 3]**

| | Feeling of use | Moisturizing feeling |
|---|---|---|
| Example 1 | ⊚ | ⊚ |
| Example 2 | ⊚ | ⊚ |
| Example 3 | ○ | ⊚ |
| Example 4 | ⊚ | △ |
| Example 5 | ○ | ⊚ |
| Example 6 | ⊚ | ⊚ |
| Example 7 | ○ | ⊚ |
| Comparative Example 1 | X | X |
| Comparative Example 2 | X | X |

Referring to Table 3 above, it can be seen that in the case of Comparative Example 1 that does not comprise vitamin B5 and Comparative Example 2 that does not comprise a neutralizer, both the feeling of use and moisturizing feeling of the cosmetic compositions are poor.

Although the preferred examples of one aspect of the present disclosure have been described in detail above, the scope of a right for one aspect of the present disclosure is not limited thereto, and various modifications and improvements made by those skilled in the art using the basic concept of one aspect of the present disclosure defined in the following claims also fall within the scope of a right for one aspect of the present disclosure.

## Claims

1. A cosmetic composition comprising polyhydroxy acid (PHA) and vitamin B5,
wherein the PHA is comprised in an amount of 15% by weight or more based on the total weight of the cosmetic composition, and
wherein the vitamin B5 is used for stabilizing PHA by preventing dissociation of PHA.

2. The cosmetic composition according to claim 1, wherein the vitamin B5 is comprised in an amount of 4 to 6% by weight based on the total weight of the cosmetic composition.

3. The cosmetic composition according to claim 1, wherein the pH of the cosmetic composition is 3.9 or more.

4. The cosmetic composition according to claim 1, wherein the pH of the cosmetic composition is maintained at 3.9 or more for 4 weeks or more.

5. The cosmetic composition according to claim 1, which does not comprise a buffer.

6. The cosmetic composition according to claim 1, wherein the cosmetic composition is formulated into a skin, lotion, cream, serum, emulsion, essence, powder, foundation, spray, sunscreen, mask pack or gel.

7. The cosmetic composition according to claim 1, further comprising one or more additives selected from the group consisting of a neutralizer, a surfactant, a moisturizer, a preservative, an ultraviolet absorber, a pigment, an inorganic salt, and an organic acid salt.

8. The cosmetic composition according to claim 7, wherein the neutralizer comprises one or more selected from the group consisting of tromethamine, sodium hydroxide, and potassium hydroxide.
